# EUROPÄISCHE PATENTSCHRIFT

(11) **EP 0 869 917 B1**
(45) Veröffentlichungstag und Bekanntmachung des Hinweises auf die Patenterteilung: **04.07.2001**
(21) Anmeldenummer: 96909038.0
(22) Anmeldetag: 11.04.1996
(51) Int. Cl.: C02F 1/30

(54) **VERFAHREN UND VORRICHTUNG ZUR BEHANDLUNG VON RECHENGUT AUS DER MECHANISCHEN REINIGUNGSSTUFE EINER KLÄRANLAGE**
PROCESS AND DEVICE FOR TREATING SCREEN FROM THE MECHANICAL CLEANING STAGE OF A SEWAGE TREATMENT PLANT
PROCEDE ET DISPOSITIF PERMETTANT DE TRAITER LES MATIERES RETENUES PROVENANT D'UN ETAGE D'EPURATION MECANIQUE D'UNE INSTALLATION DE DECANTATION D'EAUX RESIDUAIRES

(30) Priorität: 20.04.1995 DE 19514608; 20.04.1995 DE 29506702 U; 08.07.1995 DE 19524893
(43) Veröffentlichungstag der Anmeldung: 14.10.1998
(73) Patentinhaber: Egner Umwelttechnologie GmbH, 74740 Adelsheim (DE)
(72) Erfinder: Siegfried Egner, D-74740 Adelsheim (DE)
(74) Vertreter: Clemens, Gerhard, Dr.-Ing.
(86) Internationale Anmeldenummer: DE9600640
(87) Internationale Veröffentlichungsnummer: WO9633134

(56) Entgegenhaltungen:
- DE-A- 2 326 030
- US-A- 3 523 076
- US-A- 4 592 291

## Beschreibung

### TECHNISCHES GEBIET

Die vorliegende Erfindung betrifft ein Verfahren und eine Vorrichtung zur Behandlung, von Rechengut aus der mechanischen Reinigungsstufe einer Kläranlage.

Das am Ende einer mechanischen Reinigungsstufe anfallende Rechengut einer Kläranlage ist häufig mit Bakterien und Viren verseucht. Daher soll dieses Rechengut hygienisiert werden, so daß es in den nachfolgenden Behandlungsschritten beziehungsweise beim Deponieren zu keiner Gefährdung der Umwelt, insbesondere des Menschen, kommt.

### STAND DER TECHNIK

Zur Rechenguthygienisierung ist es bekannt, chemische Mittel einzusetzen, wobei sich jedoch das Problem ergibt, wie diese Mittel mit dem Rechengut ohne großen apparativen Aufwand vermischt werden können. Daher liegen immer wieder Bereiche vor, bei denen die chemischen Mittel nicht auf das Rechengut einwirken und somit in diesen Bereichen eine unzureichende Hygienisierung mit dem ihr innewohnenden Gefahrenpotential vorliegt.Eine zuverlässige Vermischung läßt sich jedoch nur mit sehr großem, in der Regel nicht vertretbarem Aufwand erzielen.

Die US-A-4595291 beschreibt ein Verfahren und eine Vorrichtung zum Behandeln von Abwasserschlamm. Dabei werden Mikrowellen eingesetzt um das Wasser aus dem Schlamm zu entfernen und anschließend werden die verbleibenden Feststoffe einer Pyrolyse zugeführt.

Die US-A-3523076 offenbart ein Verfahren zur Abwasserbehandlung, wobei das Abwasser mit Mikrowellen beaufschlagt wird. Dies soll zu einer Erhöhung der Sinkgeschwindigkeit der Feststoffeund eine Verbesserung der Filtereigenschaften des Abwassers führen.

### DARSTELLUNG DER ERFINDUNG

Der vorliegenden Erfindung liegt das technische Problem beziehungsweise die Aufgabe zugrunde, ausgehend von dem genannten Stand der Technik, ein verbessertes Verfahren und eine verbesserte Vorrichtung zur Behandlung von Rechengut aus der mechanischen Reinigungsstufe einer Kläranlage anzugeben, das beziehungsweise die mit einem wirtschaftlich vertretbaren apparativen Aufwand eine zuverlässige Hygienisierung bis hin zur Abtötung von Viren gewährleistet und wirtschaftlich ein gesetzt werden kann.

Das erfindungsgemäße Verfahren ist durch die Merkmale des unabhängigen Anspruchs 1 gegeben: Die erfindungsgemäße Vorrichtung ist durch die Merkmale des unabhängigen Anspruchs 4 gegeben. Vorteilhafte Ausgestaltungen und Weiterbildungen sind Gegenstand von abhängigen Ansprüchen.

Das erfindungsgemäße Verfahren zeichnet sich demgemäß dadurch aus, daß im Rahmen einer Hygienisierungsstufe das Rechengut mit elektromagnetischen Wellen im Frequenzbereich zwischen 1 GHz (Gigahertz) und 1 THz (Terahertz), sogenannte Mikrowellen, über eine vorgebbare Zeitdauer beaufschlagt wird, das Rechengut während und/oder nach der Behandlung einem Luftstrom ausgesetzt wird, wodurch eine konvektive Trocknung des Rechenguts erfolgt und eine durchlaufende Luftführung oder eine Kreislaufluftführung vorgenommen wird und das Rechengut vor der Hygienisierungsstufe gewaschen und/oder gepreßt wird und nach der Hygienisierungsstufe getrocknet und/oder kompaktiert wird. Dabei erfolgt die Beaufschlagung am Ende der Reinigungsstufe, so daß lediglich das Rechengut selbst hygienisiert wird und nicht das zu Anfang der Reinigungsstufe in dem Schlamm-Feststoff-Gemisch vorhandene Wasser mithygienisiert wird, da die in diesem Wasser vorhandene "Biologie" für den weiteren Klärprozeß erhalten werden soll. Der Trocknungsprozeß führt zu einer Gewichtsreduzierung des anschließend weiter zu transportierenden Rechengut. Weiterhin führt das trockene Rechengut zu einer geringeren Verschmutzung der nach der Trocknung mit dem Rechengut in Kontakt kommenden Gerätschaften.

Bevorzugt werden Mikrowellen eingesetzt, die im Frequenzbereich zwischen 2,425 und 2,475 GHz (Gigahertz) liegen, welcher Bereich auch üblicherweise bei einem Mikrowellenherd verwendet wird. Beim Hygienisiervorgang wird das Rechengut in einem von einem Gehäuse gebildeten Hohlraum durch die Einwirkung der Mikrowellen erwärmt, wobei die Mikrowellen von einem Magnetron in den Hohlraumresonator eingespeist werden. Durch dielektrische Verluste im Rechengut wird dem Mikrowellenfeld Energie entzogen und das Rechengut wird dadurch erwärmt, was bei genügend hohen Temperaturen die Abtötung von Viren zur Folge hat.

Eine bevorzugte Ausführungsvariante, die insbesondere hinsichtlich einer günstigen Energiebilanz vorteilhaft eingesetzt wird, zeichnet sich dadurch aus, daß das Rechengut in einer Aufwärmphase mit einem Magnetfeld mit hoher Energiedichte und in einer Warmhaltephase mit einem Magnetfeld mit geringerer Energiedichte beaufschlagt wird.

Die erfindungsgemäße-Vorrichtung zur Behandlung von Rechengut aus der mechanischen Reinigungsstufe einer Kläranlage mit einem Rechenguteintragsbereich und einem Rechengutaustragsbereich ist dadurch gekennzeichnet, daß eine elektromagnetische Wellen erzeugende Einrichtung, sogenannte Mikrowelleneinrichtung, vorhanden ist, die elektromagnetische Wellen im Frequenzbereich von 1 GHz (Gigahertz) bis 1 THz (Terahertz) erzeugt und das Rechengut damit über eine vorgebbare Zeitdauer beaufschlagt und eine Luftbewegungseinrichtung vorhanden ist, die das Rechengut während und/oder nach der Behandlung einem Luftstrom aussetzt, wobei bevorzugt für die Luftbewegungseinrichtung ein Niederdruckventilator eingesetzt wird. Durch das Vorsehen einer Luftbewegungseinrichtung wird ein konvektiver Trocknungsvorgang in Gang gesetzt, der eine beschleunigte Trocknung des Rechenguts zur Folge hat. Dieser Trocknungsvorgang kann sowohl in dem Reaktorraum, in dem das Rechengut erhitzt wird, als auch in einem eventuell vorhandenen Nachwärmeraum erfolgen. Bevorzugt ist vor oder hinter die Luftbewegungseinrichtung eine Filtereinheit geschaltet, um eine unangenehme Geruchsbildung außerhalb der Anlage zu verhindern. Dadurch, daß das behandelte Rechengut einen höheren Trockungsgrad aufweist, vermindert sich dessen Rohgewicht, was sich insgesamt günstig auf den anschließenden Kompaktiervorgang und den Transport zur Deponie oder Verbrennungsanlage auswirkt.

Eine vorteilhafte Ausgestaltung der erfindungsgemäßen Vorrichtung zeichnet sich dadurch aus, daß im Bereich der Einrichtung zur Erzeugung der Mikrowellenstrahlen die Vorrichtung eine Wärmeisolierung besitzt. Dies ist insbesondere hinsichtlich der einzusetzenden Energiemengen für eine zuverlässige Hygienisierung von Vorteil. In einer bevorzugten Ausführung wird das Rechengut durch eine Fördereinrichtung in den Hohlraumresonator der Mikrowelleneinrichtung verbracht.

Weitere Ausführungsformen und Vorteile der Erfindung ergeben sich durch die in den Ansprüchen ferner aufgeführten Merkmale sowie durch die nachstehend angegebenen Ausführungsbeispiele. Die Merkmale der Ansprüche können in beliebiger Weise miteinander kombiniert werden, insoweit sie sich nicht offensichtlich gegenseitig ausschließen.

### KURZE BESCHREIBUNG DER ZEICHNUNG

Die Erfindung sowie vorteilhafte Ausführungsformen und Weiterbildungen derselben werden im folgenden anhand der in der Zeichnung dargestellten Beispiele näher beschrieben und erläutert. Die der Beschreibung und der Zeichnung zu entnehmenden Merkmale können einzeln für sich oder zu mehreren in beliebiger Kombination erfindungsgemäß angewandt werden. Es zeigen:
- Fig. 1: schematische Ablaufdarstellung des Verfahrens zur Hygienisierung und
- Fig. 2: schematischer Aufbau einer Vorrichtung zur Behandlung von Rechengut einer Ausführungsvariante einer Vorrichtung zur Behandlung von Rechengut mit einer Mikrowelleneinrichtung und einer Fördereinrichtung.

### WEGE ZUM AUSFÜHREN DER ERFINDUNG

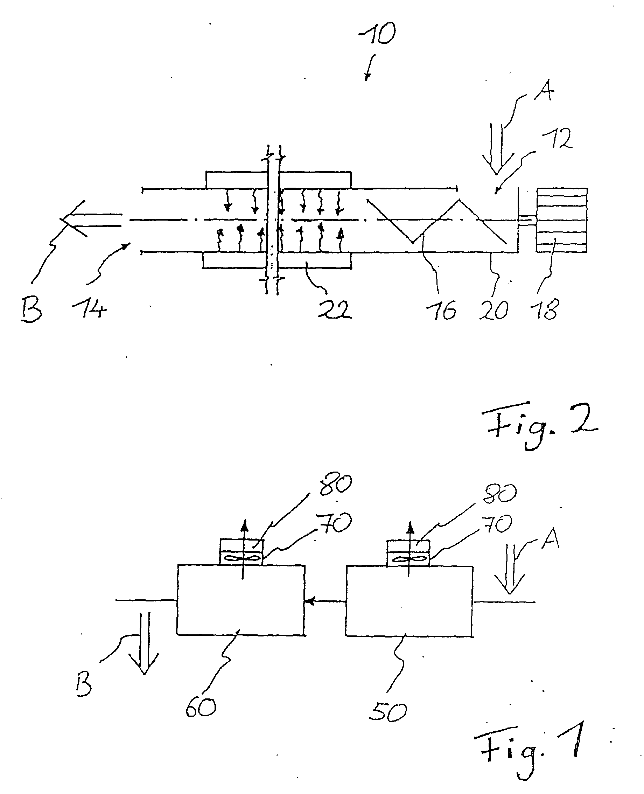

Bei dem in Fig. 1 schematisiert dargestellten Verfahrensablauf zur Hygienisierung von Rechengut wird dieses in einem ersten Verfahrensschritt einer ersten Station 50 über nicht dargestellte Fördermittel zugeführt (gemäß Pfeil A), in der das Rechengut mittels Mikrowellenstrahlung erhitzt wird. In einem zweiten Verfahrensschritt wird in einer zweiten Station 60 die Temperatur über einen vorgebbaren Zeitraum gehalten, so daß das Rechengut zuverlässig hygienisiert werden kann und die Abtötung von Viren gewährleistet werden kann. Daran anschließend wird das Rechengut (gemäß Pfeil B) ausgetragen. Die erste Station 50 und die zeite Station 60 können auch in einer einzigen Station zusammengefaßt sein, wobei dann das Rechengut nachdem es erhitzt worden ist in dieser Station eine ausreichende Zeit verweilt, innerhalb derer die erforderliche Temperatur zur Hygienisierung aufrechterhalten wird.

Die Transportmittel können als schiebende oder ziehende Transportmittel, insbesondere als Förderschnecke, ausgebildet sein. Auch ein rutschender Transport des Rechenguts ist denkbar. Die Anordnung der Transportrichtung des Rechenguts kann - wie in Fig. 1 schematisch dargestellt - horizontal sein.

Auch eine vertikale oder geneigte Transportrichtung ist problemlos umsetzbar, so daß die Bauteilkomponenten für die Hygienisierung problemlos den jeweiligen konstruktiven Platzverhältnissen angepaßt werden können.

In Fig. 1 ist schematisch oberhalb der ersten und zweiten Station eine Luftbewegungseinheit, die als Niederdruckventilator 70 ausgebildet ist, mit dem Innenraum der jeweiligen Station verbunden. Anschließend an den Niederdruckventilator 70 ist eine Filtereinheit 80 vorhanden, die verhindert, daß eine unangenehme Geruchsbildung außerhalb der Vorrichtung zustande kommt. Der Niederdruckventilator erzeugt in der Vorrichtung einen Luftstrom, der insbesondere erwärmt ist, so daß ein konvektiver Trocknungsprozeß eingeleitet wird, der zur Folge hat, daß sowohl das Gewicht des fertigbehandelten Rechengutes vermindert wird und das nach der Behandlung erforderliche weitere Verbringen des Rechengutes infolge der Trockenheit des Materials sauberer gehandhabt werden kann. Bei dem oben dargestellten Trocknungsprozeß wird die Luft im Durchlaufverfahren geführt. Es ist jedoch auch eine Luftführung im Kreislaufverfahren denkbar, was zu Energieeinsparungen führt.

Eine in Fig. 2 dargestellte Vorrichtung 10 zur Behandlung, insbesondere Hygienisierung, von Rechengut aus der mechanischen Reinigungsstufe einer Kläranlage besitzt ein Gehäuse 20, in das in einem Eintragbereich 12 gemäß Pfeilrichtung A Rechengut eingetragen wird und in einem in Fig. 1 links dargestellten Austragbereich 14 gemäß Pfeil B das Rechengut wieder ausgetragen wird. Innerhalb des Gehäuses 20 ist eine Fördereinrichtung 16 vorhanden, die als Förderschnecke ausgebildet ist und von einem außerhalb des Gehäuses 20 angeordneten Antriebsaggregat 18 angetrieben wird. Das Gehäuse 20 ist in einem vorgegebenen Bereich mit einer Mikrowelleneinrichtung 22 ausgestattet, die ihre Mikrowellenstrahlung in das Innere der Vorrichtung 10 auf das Rechengut abgibt. Die Förderschnecke transportiert das Rechengut bis zu der Mikrowelleneinrichtung 22. Dann wird das Rechengut schiebend in die Mikrowelleneinrichtung ein- und anschließend ausgebracht. Im Bereich der Mikrowelleneinrichtung 22 ist die Wandunq des Gehäuses 20 so ausgebildet, daß diese für die Mikrowellenstrahlung durchlässig ist. Das Gehäuse 20 dient im Bereich der Mikrowelleneinrichtung 22 als Hohlraumresonator, wobei die Mikrowellen selbst durch ein Magnetron in diesen Hohlraumbereich eingespeist werden. Die Erwärmung des Rechenguts und damit die Hygienisierung beziehungsweise Abtötung von Viren erfolgt dadurch, daß durch dielektrische Verluste im Rechengut dem Mikrowellenfeld Energie entzogen wird, die in Wärme umgesetzt wird.

In einer nicht dargestellten Ausführungsvariante sind im Bereich der Beaufschlagung des Rechenguts Mikrowellentemperatursensoren vorhanden, die die Temperatur im Rechengut messen. Aufgrund der gemessenen Werte, die an eine Steuereinheit abgegeben werden, wird dann die Energiedichte eingestellt, mit der das Rechengut beaufschlagt wird.

Eine zuverlässige Hygienisierung erfolgt dadurch, daß das Rechengut über eine vorgebbare ausreichende Zeitdauer mit den Mikrowellen beaufschlagt wird.

Ein umfangreiches erfindungsgemäßes Verfahren zur Behandlung von Rechengut ist durch die Kombination folgender Verfahrensschritte gegeben:
- Waschen,
- Pressen,
- Hygienisieren,
- Trocknen,
- Kompaktieren.

## Patentansprüche

1. Verfahren zur Behandlung von Rechengut aus der mechanischen Reinigungsstufe einer Kläranlage,
**dadurch gekennzeichnet**, daß
- im Rahmen einer Hygienisierungsstufe das Rechengut mit elektromagnetischen Wellen im Frequenzbereich zwischen 1 GHz (Gigahertz) und 1 THz (Terahertz), sogenannte Mikrowellen, über eine vorgebbare Zeitdauer beaufschlagt wird,
- das Rechengut während und/oder nach der Behandlung einem Luftstrom ausgesetzt wird, wodurch eine konvektive Trocknung des Rechenguts erfolgt und eine durchlaufende Luftführung oder eine Kreislaufluftführung vorgenommen wird und
- das Rechengut vor der Hygienisierungsstufe gewaschen und/oder gepreßt wird und nach der Hygienisierungsstufe kompaktiert wird.

2. Verfahren nach Anspruch 1,
**dadurch gekennzeihnet,** daß
die Mikrowellen im Frequenzbereich zwischen 2,425 und 2,475 GHz (Gigahertz) liegen.

3. Verfahren nach Anspruch 1 und/oder 2,
**dadurch gekennzeichnet,** daß
das Rechengut in einer Aufwärmphase mit einem Magnetfeld mit hoher Energiedichte und in einer Warmhaltephase mit einem Magnetfeld mit geringer Energiedichte beaufschlagt wird.

4. Vorrichtung (10) zur Behandlung von Rechengut aus der mechanischen Reinigungsstufe einer Kläranlage, mit einem Rechenguteintragbereich (12) und einem Rechengutaustragbereich (14),
**dadurch gekennzeichnet,** daß
- eine elektromagnetische Wellen erzeugende Einrichtung (22), sogenannte Mikrowelleneinrichtung (22), vorhanden ist, die elektromagnetische Wellen im Frequenzbereich von 1 GHz (Gigahertz) bis 1 THz (Terahertz) erzeugt und das Rechengut damit über eine vorgebbare Zeitdauer beaufschlagt und
- eine Luftbewegungseinrichtung (70) vorhanden ist, die das Rechengut während oder nach der Behandlung einem, insbesondere erwärmten, Luftstrom aussetzt, wobei die Luft durchlaufend oder im Kreislauf geführt wird.

5. Vorrichtung nach Anspruch 4,
**dadurch gekennzeichnet,** daß
im Bereich der Einrichtung (22) die Vorrichtung eine wärmeisolierende Wandung aufweist.

6. Vorrichtung nach Anspruch 4 und/oder 5,
**dadurch gekennzeichnet,** daß
die Vorrichtung zumindest einen Temperatursensor besitzt zum Messen der Temperatur des mit den elektromagnetischen Wellen beaufschlagten Rechenguts.

7. Vorrichtung nach einem oder mehreren der Ansprüche 4 bis 6,
**dadurch gekennzeichnet,** daß
zwischen Eintragbereich und Austragbereich eine Fördereinrichtung vorhanden ist.

8. Vorrichtung nach Anspruch 1,
**dadurch gekennzeichnet,** daß
die Luftbewegungseinrichtung (70) als Niederdruckventilator ausgebildet ist.

## Claims

1. A process for treating screenings from the mechanical cleaning stage of a sewage treatment plant, wherein,
- within the framework of a disinfection stage, the screenings have electromagnetic waves in the frequency range between 1 GHz (gigahertz) and 1 THz (terahertz), so-called microwaves, applied to them for a predeterminable period,
- during and/or after the treatment the screenings are subjected to an airstream, which results in convective drying of the screenings, and the air is guided through in one pass or in a circulation, and
- the screenings are washed and/or pressed before the disinfection stage and are compacted after the disinfection stage.

2. The process as claimed in claim 1, wherein the microwaves lie in the frequency range between 2.425 and 2.475 GHz (gigahertz).

3. The process as claimed in claim 1 and/or 2, wherein, in a heating-up phase, the screenings have a magnetic field with a high energy density applied to them and, in a heat-maintaining phase, have a magnetic field with a low energy density applied to them.

4. A device (10) for treating screenings from the mechanical cleaning stage of a sewage treatment plant, having a screenings inlet area (12) and a screenings outlet area (14), wherein
- a device (22) generating electromagnetic waves, a so-called microwave device (22), is provided, which generates electromagnetic waves in the frequency range from 1 GHz (gigahertz) to 1 THz (terahertz) and applies these to the screenings for a predeterminable period, and
- an air movement device (70) is provided, which subjects the screenings to an airstream, particularly a heated airstream, during or after the treatment, the air being guided through in one pass or in a circulation.

5. The device as claimed in claim 4, wherein the device has a heat-insulating wall in the area of the device (22).

6. The device as claimed in claim 4 and/or 5, wherein the device has at least one temperature sensor to measure the temperature of the screenings to which the electromagnetic waves are applied.

7. The device as claimed in one or more of claims 4 to 6, wherein there is a conveying device between inlet area and outlet area.

8. The device as claimed in claim 4, wherein the air movement device (70) is designed as a low-pressure fan.

## Revendications

1. Procédé pour le traitement de matières retenues provenant de l'étage d'épuration mécanique d'une installation de décantation,
caractérisé par le fait que,
- dans le cadre d'un palier d'aseptisation, les matières retenues sont sollicitées, durant un laps de temps pouvant être préétabli, par des ondes électromagnétiques situées dans la plage de fréquence comprise entre 1 GHz (gigahertz) et 1 THz (térahertz), ce qu'on appelle des "micro-ondes",
- lesdites matières retenues sont exposées à un courant d'air pendant et/ou après le traitement, de sorte qu'un séchage desdites matières retenues a lieu par convection, et qu'il s'opère un guidage d'air par circulation traversante ou un guidage d'air par circulation en boucle, et
- les matières retenues sont lavées et/ou pressées avant le palier d'aseptisation, et sont compactées après ledit palier d'aseptisation.

2. Procédé selon la revendication 1,
caractérisé par le fait que
les micro-ondes se situent dans la plage de fréquence comprise entre 2,425 et 2,475 GHz (gigahertz).

3. Procédé selon la revendication 1 et/ou 2,
caractérisé par le fait que
les matières retenues sont sollicitées, durant une phase de réchauffage, par un champ magnétique à forte densité d'énergie et, durant une phase d'entretien de chaleur, par un champ magnétique à faible densité d'énergie.

4. Dispositif (10) pour le traitement de matières retenues provenant de l'étage d'épuration mécanique d'une installation de décantation, comprenant une zone (12) d'introduction de matières retenues et une zone (14) d'évacuation des matières retenues,
caractérisé
- par la présence d'un système (22) générateur d'ondes électromagnétiques, ce qu'on appelle un "système (22) à micro-ondes" qui engendre des ondes électromagnétiques situées dans la plage de fréquence comprise entre 1 GHz (gigahertz) et 1 THz (térahertz), et sollicite ainsi les matières retenues durant un laps de temps pouvant être préétabli, et
- par la présence d'un système (70) de brassage d'air qui expose les matières retenues à un courant d'air notamment réchauffé, pendant ou après le traitement, l'air étant guidé par circulation traversante ou par circulation en boucle.

5. Dispositif selon la revendication 4,
caractérisé par le fait que
ledit dispositif présente une paroi thermiquement isolante dans la région du système (22).

6. Dispositif selon la revendication 4 et/ou 5,
caractérisé par le fait que
ledit dispositif possède au moins une sonde thermométrique en vue de mesurer la température des matières retenues sollicitées par les ondes électromagnétiques.

7. Dispositif selon l'une ou plusieurs des revendications 4 à 6,
caractérisé par le fait
qu'un système de convoyage est présent entre la zone d'introduction et la zone d'évacuation.

8. Dispositif selon la revendication 4,
caractérisé par le fait que
le système (70) de brassage d'air est réalisé sous la forme d'un ventilateur à basse pression.
